# EUROPEAN PATENT SPECIFICATION

(11) **EP 3 714 928 B1**
(45) Date of publication and mention of the grant of the patent: **19.11.2025**
(21) Application number: 18881161.6
(22) Date of filing: 22.11.2018
(51) Int. Cl.: A61M 15/00

(54) **NOVEL DRY POWDER INHALATION DEVICE**
NEUER TROCKENPULVERINHALATOR
NOUVEAU DISPOSITIF D'INHALATION DE POUDRE SÈCHE

(30) Priority: 23.11.2017 CN 201711177647
(43) Date of publication of application: 30.09.2020
(73) Proprietor: Chia Tai Tianqing Pharmaceutical Group Co., Ltd., Lianyungang, Jiangsu 222062 (CN)
(72) Inventor: LI, Changhui, Lianyungang, Jiangsu 222062 (CN); DONG, Ping, Lianyungang, Jiangsu 222062 (CN); ZHU, Xuebing, Lianyungang, Jiangsu 222062 (CN)
(74) Representative: Schulz, Oliver Frank Michael
(86) International application number: PCT/CN2018/116942
(87) International publication number: WO 2019/101135

(56) References cited:
- WO-A1-2006/101975
- WO-A1-2007/132217
- WO-A1-2011/149436
- WO-A1-2016/174393
- WO-A1-2017/079397
- CN-A- 105 920 709
- CN-A- 106 031 810
- CN-A- 106 924 845
- CN-U- 204 501 970
- CN-U- 208 389 129
- US-A- 5 901 883
- US-A1- 2009 165 791
- US-A1- 2012 145 150

## Description

### TECHNICAL FIELD

The invention belongs to the field of medical device, and relates to a new dry powder inhaler, especially relates to a capsule dry powder inhaler.

### BACKGROUND

The use of dry powder inhalers (DPI) for the treatment of bronchiectasis is well known, wherein the DPI is usually driven by the patient's respiration and disperses the drug into inhalable powder by aerodynamic method.

The capsule dry powder inhaler is a well-known dry powder inhaler, which comprises a capsule chamber and an actuator for opening the capsule chamber. The opening of the capsule is mainly achieved by shearing force, acupuncture or cutting, wherein the acupuncture is the most common opening mechanism, such as the capsule inhaler disclosed in US 8196578 B2.

For the capsule DPI of the acupuncture mechanism, the powder contained in the capsule is released by piercing the capsule during atomization. When the patient inhales to generate sufficient airflow, the capsule starts to rotate and vibrate in the capsule chamber. As the inspiratory flow increases, the rotate speed of the capsule will increase, thereby generating sufficient centrifugal force to release the powder from the capsule. Therefore, the capsule DPI also has the problem of insufficient inspiratory flow for patients with impaired ability to produce sufficient inspiratory flow, and is generally not recommended for children under 5 and patients with impaired respiratory function.

The patent application CN204501970U relates to a dry powder inhaler, including the flat runner cavity of putting, runner cavity front end is equipped with the suction nozzle, runner cavity end is equipped with the back cover, be equipped with the impeller in the runner cavity, the impeller is connected with the back cover through put down and put the shaft, the shaft front end is equipped with directional suction nozzle and is used for the fixed flat capsule mounting groove of putting capsule, runner cavity lateral wall is equipped with the two and is used for the flat puncture subassembly of putting capsule of puncture, the puncture subassembly includes: swing joint embeds reset spring's the briquetting of pressing in runner cavity lateral wall to and run through runner cavity lateral wall and point to the flat pjncture needle of putting capsule, the pjncture needle is fixed in to be pressed on the briquetting to put capsule by putting down in order to puncture according to the briquetting drive, the back cover is equipped with a plurality of inlet ports, also be equipped with in the runner cavity and erect the screen cloth of putting, the screen cloth is located between suction nozzle and the capsule mounting groove. The utility model discloses dry powder inhaler can reduce the deposit of medicine granule, improves the medicine granule and sends efficiency through the lung.

The patent application US2009/165791A1 relates to an inhaler for administering a medicament in the form of inhalable substances, substance formulations, or substance mixtures, especially in the form of a powder. Said inhaler comprises a housing (1) which is provided with an interior (18) for accommodating at least one capsule that is filled with the medicament. The housing (1) is coupled to a mouthpiece (5) while an actuator (12) encompassing at least one needle (13) is mounted so as to be movable towards the housing in order to open the capsule. The mouthpiece (5) is effectively connected to the actuator (12) in such a way that the needle (13) of the actuator (12) does not extend into the interior (18) for 16 puncturing the capsule in an operational position of the mouthpiece (5).

The patent application WO2011/149436A1 relates to an inhaler which is suitable for delivering medicament in dry powder form used in prophylaxis and treatment of respiratory diseases from a capsule and comprises a deagglomerating member contributing to an effective inhalation.

The patent application WO2017/079397A1 relates to a device for the delivery of a dry powdered or aerosolized substance (e.g., medication) to a user via inhalation. Delivery of the dry powdered substance is performed using an inhalation device having an inhalation tube and a hollow extension housing an assembly of components including an impeller, a puncturing device and a dry powder container. The inhalation tube, extension and assembly are configured such that a user inhaling a breath from an end of the inhalation device creates a negative pressure on the container of dry powdered substance thereby drawing the dry powdered substance from the container into the user's lungs. The delivery of the dry powder through the inhalation device is facilitated by the puncturing device that punctures the dry powder container, the impeller that directs the flow of air and the particular configuration of the extension and the inhalation tube.

The patent application WO2007/132217A1 relates to a dry powder inhaler for pulmonary or nasal use, comprising at least an inhaler body (100) and a cartridge (110) with one or two compartments (121) each containing one dose of a drug. The compartment (121) has holes (123) to admit air and holes (120) to deliver powder, which, in use, communicate with an inhalation (102) channel in the inhaler body (100). To prevent the powder from leaking through the compartment holes before use, the air admission holes (123) are of reduced dimension, thereby blocking or hindering powder exit under the force of gravity. When the cartridge 110 is inserted inside the inhaler body (100), all its holes (120, 123) are blocked. By sliding the cartridge (110) in relation to the body (100) until the inhalation position is reached, the holes (120, 123) come into in fluid communication with one another and with the inhalation channel (103), thereby allowing a flow of air to disperse and entrain the dose through the mouthpiece (103). Five embodiments of the invention are disclosed.

WO2007/132217A1 refers to a dry powder inhaler for pulmonary or nasal use. In addition, combination therapies involving different and complementary active ingredients are also known. Currently, not only two, but also three or four active ingredients combination therapies have emerged. Although combination products provide additional convenience for patients, some pharmaceutical active ingredients are difficult to formulate into a single combination product. For example, when formulated together, the active ingredients may chemically interact with each other to have a negative effect.

US 2012/145150 A1 discloses another example of a powder inhaler.

The applicant has found that a capsule dry powder inhaler provides an effective method to solve the above-mentioned problems. The capsule dry powder inhaler comprises at least two capsule chambers and actuators matching the number of capsule chambers, each capsule chamber is loaded with capsules containing different pharmacologically active ingredients. By mixing the powder released from the capsules in different capsule chambers, a combined inhalation medicinal product can be provided to the patient.

### SUMMARY

The invention is set out in the appended set of claims.

First, the present disclosure relates to a powder release device for inhalation administration comprising:
a capsule chamber, which is a cylindrical chamber that can hold the capsule upright, and the top of the capsule chamber is open;
an actuator, which comprises a needle, mounted to be movable toward the side wall of the capsule chamber to puncture the capsule, and at least one part of the actuator is located outside of the dry powder inhaler for manipulation by the user;
a nozzle, with an outlet duct extending from the top to the bottom, a screen cover is mounted at the bottom of the outlet duct, the screen cover is embedded with a screen, and detachably connected to the top of the capsule chamber to cover the top of the capsule chamber;
wherein, the capsule chamber is provided at its bottom and / or side wall with a deflected intake duct group ventilating with the outside air, the deflected intake duct group comprises at least two deflected intake ducts which are arranged around the central axis of the capsule chamber, simultaneously deflect clockwise or counterclockwise, in order to provide a spiral airflow that flows upward from the deflected intake ducts when the user inhales.

Preferably, the deflected intake ducts of the deflected intake duct group have the same shape and size, and are evenly arranged around the central axis of the capsule chamber.

Preferably, the lower side of the screen protrudes toward the capsule chamber.

Preferably, the bottom of the capsule chamber is provided with intake ducts opened upward and ventilating with the outside air to provide air flow from bottom to top.

Preferably, the capsule chamber is provided at the bottom or the lower part of the side wall thereof with intake ducts, which are opened upward and ventilating with the outside air to provide a through airflow from bottom to top, and the capsule chamber is provided at the bottom and/or the side wall thereof with a deflected intake duct group to provide a spiral airflow rotating around the capsule chamber.

Further preferably, the intake duct at the bottom of the capsule chamber is opened upward along the central axis of the capsule chamber.

Further preferably, the intake ducts at the bottom of the capsule chamber form a deflected intake duct group.

Further preferably, the deflected intake duct group is arranged at the bottom as a fixed impeller as a whole.

Further preferably, the opening of the deflected intake ducts is tangent to the side wall of the capsule chamber.

Preferably, the side wall of the capsule chamber is provided with a deflected intake duct group.

Further preferably, the lower and / or middle part of the side wall of the capsule chamber is provided with a deflected intake duct group.

Further preferably, the lower part of the side wall of the capsule chamber is provided with a deflected intake duct group.

Further preferably, the opening of the deflected intake duct is tangent to the side wall of the capsule chamber.

Further preferably, the opening of the deflected intake duct of the side wall of the capsule chamber has a strip shape, which is arranged longitudinally along the side wall of the capsule chamber.

Further preferably, the opening of the deflected intake duct of the side wall of the capsule chamber has a strip shape parallel to the central axis of the capsule chamber.

Preferably, a deflected intake duct group composed of two deflected intake ducts is provided in the lower part and / or the middle of the side wall of the capsule chamber, and an intake duct is provided at the bottom of the capsule chamber.

Preferably, a deflected intake duct group composed of two deflected intake ducts is provided at the lower part of the side wall of the capsule chamber and a direct intake duct is provided at the bottom of the capsule chamber.

Preferably, the diameter of the capsule chamber is 1.1 to 2.5 times that of the capsule, and the height of the capsule chamber is 1.02 to 2.0 times that of the capsule.

Further preferably, the diameter of the capsule chamber is 1.2 to 1.5 times that of the capsule, and the height of the capsule chamber is 1.05 to 1.3 times that of the capsule.

In a specific embodiment of the present disclosure, the diameter of the capsule chamber is 1.35 times that of the capsule and the height of the capsule chamber is 1.15 times that of the capsule.

Preferably, the side wall of the capsule chamber is provided with a first deflected intake duct group, and the bottom of the capsule chamber is provided with a second deflected intake duct group.

In another aspect, the present disclosure relates to a method for releasing inhalable powder, comprising the following steps:
(1) Pack the capsule into a cylindrical capsule chamber that can hold the capsules upright;
(2) Cover the screen cover to the top of the capsule chamber, so that the nozzle is connected to the top of the capsule chamber through its outlet duct below it;
(3) Puncture the capsule;
(4) Inhale at the nozzle to create the following airflow in the capsule chamber: a) bottom-to-top through airflow that enters through the intake duct at the bottom of the capsule chamber or the deflected intake duct group at the lower part of the side wall and exits from the top of the capsule chamber; b) a spiral airflow that enters through the deflected intake duct group at the bottom and / or side wall of the capsule chamber and exits from the top of the capsule chamber, the through airflow and the spiral airflow together promote the rotation and vibration of the capsule in the capsule chamber to release the inhalable powder.

Preferably, in the step (4), inhale at the nozzle to create the following airflow in the capsule chamber: a) a bottom-to-top through airflow that enters through the intake duct at the bottom of the capsule chamber and exits from the top of the capsule chamber and b) a spiral airflow that enters through the deflected intake duct group through the side wall of the capsule chamber and exits from the top of the capsule chamber, the through airflow and the spiral airflow together promote the rotation and vibration of the capsule in the capsule chamber to release the inhalable powder.

Preferably, the method for releasing the inhalable powder in the capsule uses the powder release device as described in the first part.

The method for releasing inhalable powder of the present disclosure can create a spiral airflow when the user inhales, which reduces the starting flow rate of the capsule to release the inhalable powder, enables the user to provide less inspiratory flow to allow the capsule to rotate and vibrate at high speed to release the drug power, and provides a way for children under 5 and patients with impaired respiratory function to use capsule DPI.

The powder release device herein disclosed can create a spiral air flow through a deflected intake duct in the capsule chamber when the user inhales, which reduces the starting flow rate of the rotation, allows the user to provide less inspiratory flow to make the capsule rotate at high speed to release the drug, and provides a way for children under 5 years of age and patients with impaired respiratory function to use capsule DPI.

The present invention is directed to a dry powder inhaler, comprising:
capsule chambers, which are cylindrical chambers that can hold the capsule upright, the top of the capsule chambers is open, and the bottom and / or the side walls of the capsule chambers are provided with intake ducts ventilating with the outside air;
actuators, comprising puncture needles, mounted for the user to operate to move toward the side walls of the capsule chambers to puncture the capsules;
a nozzle, comprising an outlet duct, said outlet duct being positioned under the nozzle:
wherein, the number of the capsule chambers is two to four, and all the capsule chambers are arranged in parallel to form an integral multi-capsule chamber, the actuators are mounted individually or in common among the capsule chambers, and the actuators are mounted with needles in the width direction of the actuators, at least the number of the needles is same as that of the matching capsule chambers, a screen cover, said screen cover being mounted at the bottom of the outlet duct under the nozzle, and a screen, said screen being fixed in the screen cover and separately connected to the top of the multi-capsule chamber such that the screen covers the top of all of the capsule chambers; wherein the dry powder inhaler further comprises a central baffle, the central baffle being arranged such that:
   the lower part of the outlet duct is divided by said central baffle to form two sub-ducts, which are respectively connected to the top of each capsule chamber,
   a cross-section of each sub-duct being such that it first narrows from the top of each capsule chamber from bottom to top toward the central baffle, and then remains unchanged,
   wherein the sub-duct comprises one or more sub-baffles, the one or more sub-baffles dividing the sub-duct into narrower ducts, which gradually narrow from bottom to top from the top of each capsule chamber toward the central baffle.

Preferably, the side wall of the outlet duct is provided with at least one small hole ventilating with the outside air, and the small hole is opened in a direction not facing the central axis of the outlet duct to promote airflow rotate in the outlet duct when the user inhales.

Preferably, the diameter of the outlet duct gradually decreases from bottom to top, and a neck is formed before arriving at the nozzle, and the small hole of the outlet duct is provided at the lower side of the neck.

Further preferably, the number of the small holes in the side wall of the outlet duct is two, the two small holes are symmetrically opened around the central axis of the outlet duct.

Preferably, an intake duct is provided at the bottom of each capsule chamber to provide air flow from bottom to top when the user inhales. The intake duct opens upward and ventilates with outside air.

Further preferably, the intake duct at the bottom of each capsule chamber comprises a deflected intake duct group, the deflected intake duct group comprises at least two deflected intake ducts which are arranged around the central axis of the capsule chamber, simultaneously deflect clockwise or counterclockwise, in order to provide a spiral airflow that flows upward from the bottom when the user inhales.

Further preferably, the intake ducts at the bottom of each capsule chamber is a deflected intake duct group, the deflected intake duct group comprises at least two deflected intake ducts which are arranged around the central axis of the capsule chamber, simultaneously deflect clockwise or counterclockwise, in order to provide a spiral airflow that flows upward from the bottom when the user inhales.

Further preferably, the deflected intake ducts of the deflected intake duct group of each capsule chamber have the same shape and size, and are evenly arranged around the central axis of the capsule chamber.

Further preferably, the opening of the deflected intake ducts at the bottom of the capsule chamber is tangent to the side wall of the capsule chamber.

In a specific embodiment of the present disclosure, the deflected intake duct at the bottom of each capsule chamber is arranged as a fixed impeller as a whole.

Preferably, the side wall of each capsule chamber is provided with a deflected intake duct group, the deflected intake duct group comprises at least two deflected intake ducts which are arranged around the central axis of the capsule chamber, simultaneously deflect clockwise or counterclockwise, in order to provide a spiral airflow that flows upward from the bottom when the user inhales.

Further preferably, the deflected intake duct group is provided in the middle and / or lower part of the side wall of the capsule chamber.

Further preferably, the deflected intake duct group is provided at the lower part of the side wall of the capsule chamber.

Further preferably, the deflected intake ducts in a deflected intake duct group have the same shape and size, and are evenly arranged around the central axis of the capsule chamber.

Further preferably, the opening of the deflected intake duct is tangent to the side wall of the capsule chamber.

Further preferably, the opening of the deflected intake duct of the side wall of the capsule chamber has a strip shape, which is arranged longitudinally along the side wall of the capsule chamber.

Further preferably, the opening of the deflected intake duct of the side wall of the capsule chamber has a strip shape parallel to the central axis of the capsule chamber.

Further preferably, the number of deflected intake ducts of a deflected intake duct group is two.

In a specific embodiment, a deflected intake duct group composed of two deflected intake ducts is provided at the lower part of the side wall of the capsule chamber, and an intake duct is provided at the bottom of the capsule chamber.

Preferably, the size of the intake duct and / or top opening of at least one capsule chamber is different from that of other capsule chambers, so that the air flow rate in the capsule chamber is different from that of the other capsule chambers.

Further preferably, the size of the intake duct of at least one capsule chamber is different from that of other capsule chambers, so that the air flow rate in the capsule chamber is different from that of the other capsule chambers.

Preferably, the diameter of the capsule chamber is 1.1 to 2.5 times the diameter of the capsule, and the height of the capsule chamber is 1.02 to 2.0 times the height of the capsule.

Further preferably, the diameter of the capsule chamber is 1.2 to 1.5 times the diameter of the capsule, and the height of the capsule chamber is 1.05 to 1.3 times the height of the capsule.

In a specific embodiment of the present disclosure, the diameter of the capsule chamber is 1.35 times the diameter of the capsule and the height of the capsule chamber is 1.15 times the height of the capsule.

Preferably, the multi-capsule chamber is composed of a first capsule chamber and a second capsule chamber, a first actuator and a second actuator are arranged at both ends of the connecting line where the first capsule chamber and the second capsule chamber are located, the first actuator and the second actuator can move from both sides to the middle so as to puncture the capsules in the first capsule chamber and the second capsule chamber, respectively.

Preferably, the multi-capsule chamber is composed of first capsule chamber and a second capsule chamber, which are closely arranged, a first actuator and a second actuator are arranged at both ends of a line connecting the first capsule chamber and the second capsule chamber, the first actuator and the second actuator can move from both sides to the middle so as to puncture the capsules in the first capsule chamber and the second capsule chamber, respectively.

Preferably, the multi-capsule chamber is composed of first capsule chamber and second capsule chamber, which are closely arranged, and an actuator is arranged on one side of the line connecting the first capsule chamber and the second capsule chamber, the actuator comprises at least two needles in the width direction to puncture the capsules in the first capsule chamber and the second capsule chamber at the same time.

According to the invention, the lower part of the outlet duct is divided by a central baffle to form a first sub-duct and a second sub-duct, which are respectively connected to the tops of the first capsule chamber and the second capsule chamber, the first sub-duct and the second sub-duct gradually narrow from bottom to top from the top of each capsule chamber toward the central baffle, their cross-sections gradually narrow or maintain, they direct the airflows of the first capsule chamber and the second capsule chamber converge to the upper part of the outlet duct, along the first sub-duct and the second sub-duct respectively, when the user inhales. More preferably, the cross-sections of the first sub-duct and the second sub-duct gradually narrow from the top of each capsule chamber first, and then remain.

Further preferably, the cross-section of the outlet duct maintains the same size or gradually increases from the top of the central baffle toward the nozzle. Further preferably, the cross-sections of the outlet ducts gradually increase in a direction from the top of the central baffle toward the nozzle first, and then remain.

According to the invention, the first sub-duct and the second sub-duct further comprise one or more sub-baffles, the one or more sub-baffles divide the first sub-duct and the second sub-duct to narrower ducts respectively, which gradually narrow from bottom to top from the top of each capsule chamber toward the central baffle. More preferably, the height of the sub-baffle is lower than that of the central baffle. More preferably, the whole shape of the cross-section of the sub-baffles is like "X", which takes the central baffle as the plane mirror symmetry.

Further preferably, the nozzle is mounted at where the airflow from the first capsule chamber and the second capsule chamber has fully converged in the upper part of the outlet duct along the first sub-duct and the second sub-duct respectively when the user inhales.

More preferably, the length of the outlet duct is 25-36 mm, most preferably 31 mm.

Further preferably, the air resistance of the dry powder inhaler is 0.01-0.08KPa ^{0.5} minutes / liter.

More preferably, the air resistance of the dry powder inhaler is 0.02-0.05KPa ^{0.5} minutes / liter.

In a specific embodiment, the air resistance of the dry powder inhaler is 0.0325KPa ^{0.5} minutes / liter.

Preferably, the multi-capsule chamber is closely arranged into a triangle by the first capsule chamber, the second capsule chamber and the third capsule chamber, and a first actuator is arranged on one side of the line connecting the first capsule chamber and the second capsule chamber, the first actuator comprises at least two needles in the width direction to puncture the capsules in the first capsule chamber and the second capsule chamber at the same time, a second actuator is arranged on the side of the third capsule chamber that far away from the first capsule chamber and the second capsule chamber, it is movable in a vertical direction of the straight line where the first capsule chamber and the second capsule chamber are located to puncture the capsule in the third capsule chamber .

Further preferably, the air resistance of the dry powder inhaler is 0.015-0.073KPa^{0.5} minutes / liter.

More preferably, the air resistance of the dry powder inhaler is 0.02-0.04KPa ^{0.5} minutes / liter.

In a specific embodiment of this application, the air resistance of the dry powder inhaler is 0.0305KPa ^{0.5} minutes / liter.

Preferably, the multi-capsule chamber is closely arranged into a square by the first capsule chamber, the second capsule chamber, the third capsule chamber and the fourth capsule chamber, and a first actuator and a second actuator are arranged on the central axes of the square and movable from both sides to the middle, the first actuator and the second actuator comprise at least two needles in the width direction, so that the first actuator punctures the capsules in the capsule chamber and the second capsule chamber at the same time, the second actuator punctures the capsules in the third capsule chamber and the fourth capsule chamber at the same time.

Further preferably, the air resistance of the dry powder inhaler is 0.01-0.06KPa ^{0.5} minutes / liter.

Even more preferably, the air resistance value of the dry powder inhaler is 0.015-0.035KPa^{0.5} minutes / liter.

In a specific embodiment of the present disclosure, the air resistance of the dry powder inhaler is 0.029KPa ^{0.5}minutes / liter.

Preferably, the dry powder inhaler comprises:
a lower casing, which defines a cavity, the tope of which is open and is used for accommodating a multi-capsule chamber inside, the side of the lower casing is provided with gaps that match the number and position of the actuators, so that part of each actuator is located outside the dry powder inhaler for the user to operate, and the cavity ventilates with the outside air;
an adapter plate, which covers the top of the lower casing, and a hollow port is provided at the top of the multi-capsule chamber, a screen cover is detachably mounted to the hollow port so that the screen can cover the top of each capsule chamber through the hollow port;
an upper casing, which extends downward from the top of the nozzle, defines a cavity surrounding the outlet duct and the bottom of the cavity being open, and covers the adapter plate when the screen cover is mounted at the hollow port.

In an example of the present disclosure,
the dry powder inhaler comprises:
a lower casing, which defines a cavity, the tope of which is open and is used for accommodating a multi-capsule chamber inside, the side of the lower casing is provided with gaps, the number and position of which match with the number and position of the actuators, so that at least one part of each actuator is located outside the dry powder inhaler for the user to operate, and the cavity ventilates with the outside air through an air intake hole provided on the side and / or at the bottom of the lower casing;
a adapter plate, which covers the top of the lower casing, and a hollow port is provided at the top of the multi-capsule chamber, a screen cover is detachably mounted to the hollow port so that the screen can cover the top of each capsule chamber through the hollow port;
an upper casing, which extends downward from the top of the nozzle, defines a cavity surrounding the outlet duct and the bottom thereof being open, and covers the adapter plate when the screen cover is mounted at the hollow port.

Preferably, the multi-capsule chamber is formed integrally with the adapter plate, which is fixed below the hollow port of the adapter plate.

Preferably, the gaps are widened and / or extended based on the size of the actuator to provide the air intake hole.

Preferably, the side wall of the outlet duct is provided with at least one small hole ventilating with the outside air, and the small hole is opened in a direction not facing the central axis of the outlet duct to promote airflow rotate in the outlet duct when the user inhales.

Preferably, the upper casing, the lower casing and the adapter plate are connected together by a hinge on the same side.

Preferably, the adapter plate and the capsule chamber are integrally formed, and the hollow port constitutes the top opening of each capsule chamber.

Preferably, the diameter of the outlet duct gradually decreases from bottom to top, and a neck is formed before arriving at the nozzle.

Preferably, a slit or a hole is provided at the junction of the upper casing and the adapter plate, so that the cavity can ventilate with the outside air through the slit or the hole;

Further preferably, the number of small holes on the outlet duct is two, the two small holes are symmetrically opened around the central axis of the outlet duct.

Further preferably, the small holes on the outlet duct are located in the area below the neck.

The dry powder inhaler of this application provides a medicine dispenser containing different active components (or a mixture thereof) separately by providing a plurality of capsule chambers arranged in parallel. The structure is simple and the operation is convenient. In addition, each capsule chamber can adjust the parameters of the intake duct and the air exhaust duct according to the nature of the powder of the drug (composition), in order to provide suitable particle distribution for each active ingredient.

### BRIEF DESCRIPTION OF THE DRAWINGS

FIG. 1 shows a disassembled view of the structure of a powder release device.
FIG. 2 shows a sectional view of a capsule chamber of the powder release device shown in FIG.1
FIG. 3 shows a cross-sectional top view of the capsule chamber shown in FIG. 2.
FIG. 4 shows another sectional view of the capsule chamber of the powder release device shown in FIG. 1.
FIG. 5 shows a structural view of the lower part of the section of the capsule chamber shown in FIG. 4.
FIG. 6 shows another sectional view of the capsule chamber of the powder release device shown in FIG. 1.
FIG. 7 shows a sectional top view of the capsule chamber shown in FIG. 6.
FIG. 8 shows another sectional view of the capsule chamber area of the powder release device shown in FIG. 1.
FIG. 9 shows a sectional top view of the capsule chamber shown in FIG. 8.
FIG. 10 is a partially enlarged view of the impeller at the bottom of the capsule chamber shown in FIG. 9.
FIG. 11 is a sectional view of another capsule chamber area of the powder release device shown in FIG. 1.
FIG. 12 shows a sectional top view of the capsule chamber area shown in FIG. 11.
FIG. 13 shows a disassembled view of the structure of a dry powder inhaler.
FIG. 14 is a partial cross-sectional view of the dry powder inhaler shown in FIG. 13.
FIG. 15 is a perspective view of the nozzle of the dry powder inhaler shown in FIG. 13.
FIG. 16 is a perspective view of the screen cover of the dry powder inhaler shown in FIG. 13.
FIG. 17 shows a sectional plan view of the multi-capsule chamber of the dry powder inhaler shown in FIG. 13.
FIG. 18 shows a sectional top view of a multi-capsule chamber of a possible dry powder inhaler.
FIG. 19 shows a sectional top view of a multi-capsule chamber of another possible dry powder inhaler.
FIG. 20 is a partial sectional view of another dry powder inhaler of the present invention.
FIG. 21 is a sectional view of the airflow of the nozzle of the dry powder inhaler shown in FIG. 20.
FIG. 22 is a plan view of the nozzle of the dry powder inhaler shown in FIG. 20.

### DETAILED DESCRIPTION OF THE EMBODIMENTS

The present invention will be further described below in conjunction with specific embodiments.

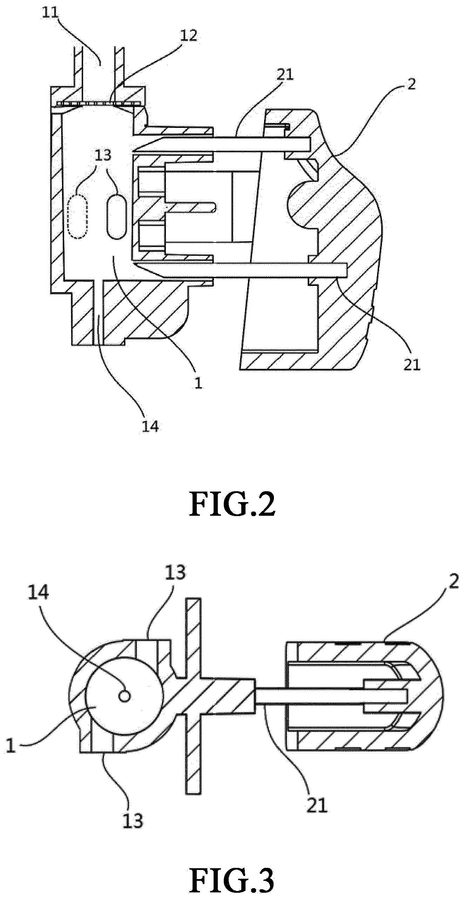

By referring to FIG. 1, FIG. 2 and FIG. 3, FIG. 1 illustrates a specific embodiment of a powder release device of the present disclosure, comprising: (a) a capsule chamber 1, which is a cylindrical chamber that can receive the capsule, the capsules chamber 1 is provided with an outlet duct 11 at the top thereof, and a ventilating screen 12 is mounted at the junction of the outlet duct 11 and the capsule chamber 1; (b) the actuator 2, which comprises at least one needle 21, which is mounted as it can move to the capsule chamber 1 to puncture the capsule, and at least one part of the actuator 2 is located outside the powder release device for the user to manipulate; (c) the nozzle 3, which is connected to the top of the capsule chamber 1 through the outlet duct 11.By referring to FIG. 2, the capsule chamber 1 is provided with a deflected intake duct group that ventilates with outside air. By referring to, the deflected intake duct group comprises at least two deflected intake ducts 13, which are arranged around the central axis of the capsule chamber and simultaneously deflect clockwise or counterclockwise, in order to provide a spiral airflow that flows from the deflected intake duct group to the top outlet duct 11 when the user inhales.

In this embodiment, the user opens the screen 12 mounted above the capsule chamber 1 first and puts the capsule herein, then presses the actuator 2 to puncture the capsule, and the actuator 2 is then reset by manual operations or elastic means. Since the nozzle 3 ventilates with the capsule chamber 1 through an outlet duct 11, and the capsule chamber 1 ventilates with the external environment through the deflected intake duct group, when the user inhales, the outside air passes through the deflected intake duct group to generate a spiral airflow surrounding the capsule chamber 1, to promote a rapid rotation of the punctured capsule to release the inhalable medicinal powder contained therein. The inhalable medicinal powder moves with the airflow to the outlet duct 11 at the top of the capsule chamber 1 and enters the user's body through the nozzle 3.

It should be noted that the deflected intake duct 13 of this embodiment is deflected clockwise or counterclockwise, but it does not mean that the deflected intake duct group must be opened in the horizontal direction, as long as it can provide a part of the air flow deflected in the horizontal direction. Of course, the at least two deflected intake ducts 13 should be deflected simultaneously. For example, when the deflected intake passages 13 are located on the side wall, they should all face diagonally upward, all face diagonally downward, or all face horizontally.

Compared with the prior art, the powder release device of this embodiment provides the deflected intake duct group in the capsule chamber 1, so that the requirement of the user's inhalation flow when the capsule rotates and releases medicine is greatly reduced, the powder is easier to release and the amount of residue reduces.

The deflected intake ducts 13 of the deflected intake duct group have the same shape and size, and are evenly arranged around the central axis of the capsule chamber 1 to provide more uniform spiral airflow. Since the release of the inhalable powder in the capsule is achieved by the rotation and vibration of the capsule in the capsule chamber 1, but not based on only the rotation, the shapes and sizes of the deflected intake ducts 13 of the deflected intake duct group do not have to be exactly the same.

Compared with the prior art, the powder release device of this embodiment provides the deflected intake duct group in the capsule chamber 1, so that the requirement of the user's inhalation flow rate when the capsule rotates and vibrates to release medicine is greatly reduced, the powder is easier to release and the amount of residue is reduced.

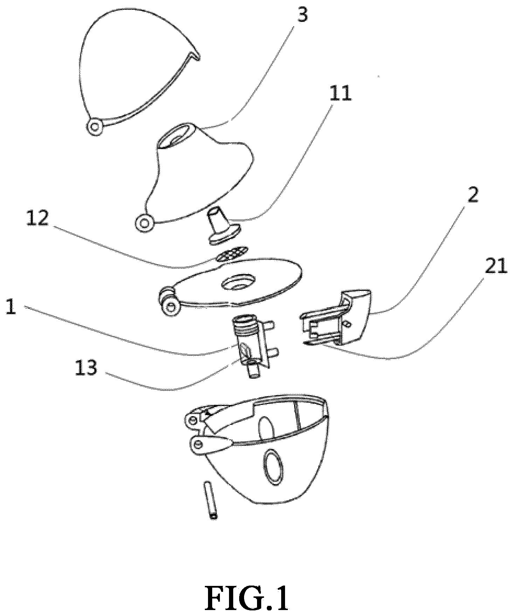

Further preferably, by referring to FIG. 1, in one embodiment, the lower side of the ventilating screen 12 protrudes toward the capsule chamber 1, and this shape can provide a capsule rotating contact surface with less resistance.

Further preferably, by referring to FIG. 2 and FIG.3, in one embodiment, the deflected intake duct group is provided on the side wall of the capsule chamber 1, and the bottom of the capsule chamber 1 is also provided with an intake duct 14 ventilating with the outside air, which is opened upwards along the central axis of the capsule chamber 1 to provide an air flow throughout the capsule chamber 1 from bottom to top when the user inhales.

In this embodiment, the intake duct 14 at the bottom of the capsule chamber 1 can provide a through airflow throughout the entire capsule chamber 1 from bottom to top when the user inhales, in order to help the top of the capsule to rotate against the ventilating screen 12 at the top of the capsule chamber 1, so that the powder released from the capsules moves toward the top of the outlet duct 11 more smoothly.

In this embodiment, the opening of the deflected intake duct 13 at the side wall of the capsule chamber 1 has a strip shape, which is arranged longitudinally along the side wall of the capsule chamber, in order to provide an airflow having a larger surface contact with the capsule when the user inhales, so as to drive the capsule to rotate and vibrate easier in the capsule chamber 1 to release the inhalable powder.

Further preferably, by referring to FIG. 2 and FIG. 3, in one embodiment, the opening of the deflected intake duct 13 at the side wall of the capsule chamber 1 has a strip shape which is parallel to the central axis of the capsule chamber, so that when the user inhales, an airflow with a larger surface contact with the capsule and a better applying force direction is provided to drive the capsule to rotate and vibrate more smoothly in the capsule chamber 1 so as to release the inhalable powder.

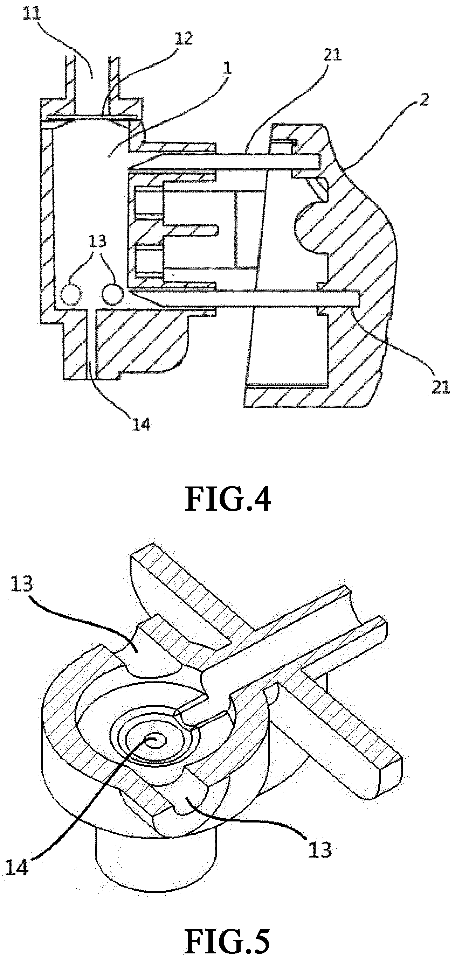

Further preferably, by referring to FIG. 4 and FIG.5, in one embodiment, the deflected intake duct group is provided at the lower part of the side wall of the capsule chamber 1, and the intake duct 14 is provided at the bottom of the capsule chamber 1, wherein the intake duct is opened upwards along the central axis of the capsule chamber 1 to provide an air flow throughout the capsule chamber 1 from bottom to top when the user inhales.

The deflected intake duct group is provided at the lower part of the side wall of the capsule chamber 1, which can better provide a through airflow throughout the entire capsule chamber 1 from bottom to top when the user inhales, in order to help the top of the capsule to rotate against the ventilating screen 12 at the top of the capsule chamber 1, so that the powder released from the capsule moves toward the top of the outlet duct 11 more smoothly.

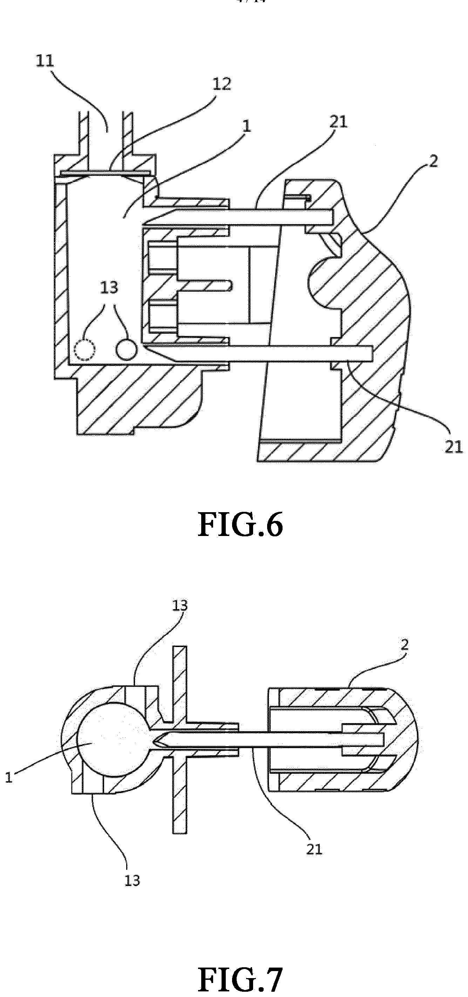

Further preferably, by referring to FIG. 6 and FIG. 7, in one embodiment, the deflected intake duct group is provided at the lower part of the side wall of the capsule chamber 1, and the intake duct 14 is not provided at the bottom of the capsule chamber 1.

The deflected intake duct group is only provided at the lower part of the side wall of the capsule chamber 1, which can also provide a bottom-up airflow when the user inhales, in order to help the top of the capsule to rotate against the ventilating screen 12 at the top of the capsule chamber 1. It can be understood that, in order to make the top of the capsule rotate against the screen 12 at the top of the capsule chamber 1, the through airflow is not necessary, as long as a non-through airflow from bottom to top is enough to lift the capsule.

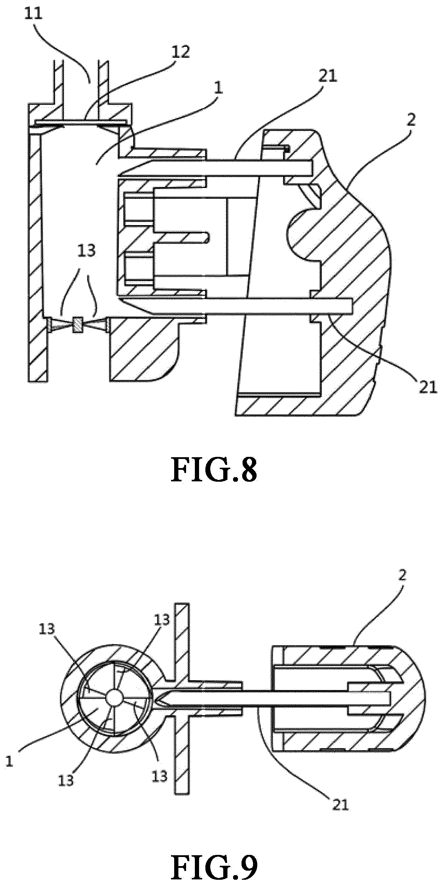

By referring to FIG. 8, FIG. 9 and FIG. 10, in another embodiment of a powder release device of the present disclosure, a deflected intake duct group is provided at the bottom of the capsule chamber 1.

The deflected intake duct group at the bottom of the capsule chamber 1 can also be evenly arranged around the central axis of the capsule chamber, simultaneously deflect clockwise or counterclockwise. When the user inhales, it can provide a part of the spiral airflow that is deflected in the horizontal direction to help the capsule rotate and vibrate to release the inhalable powder, and it can also provide another part of the bottom-to-up through airflow to help the top of the capsule rotate against the ventilating screen 12 at the top of the capsule chamber 1, so that the powder released from the capsule moves towards the top of the outlet duct 11 more smoothly. The structure is simple, and it serves two purposes.

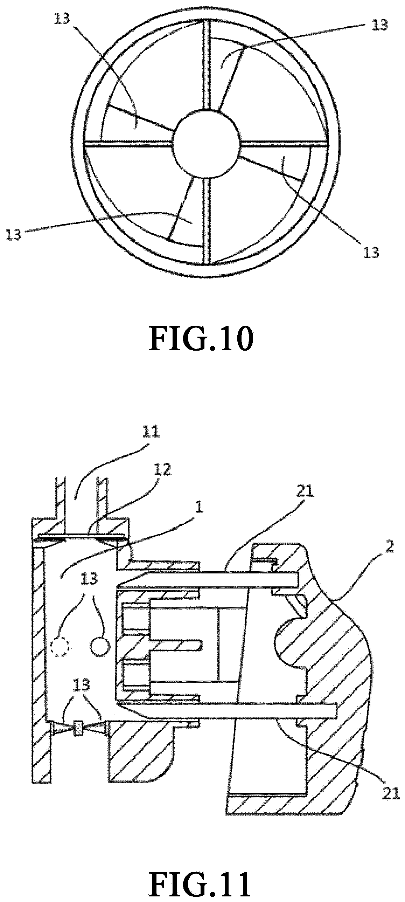

Further preferably, by referring to FIG. 9 and FIG. 10, in one embodiment, the deflected intake duct group at the bottom is arranged as a fixed impeller as a whole.

In this embodiment, the deflected intake duct group of the impeller structure at the bottom of the capsule chamber 1 can be understood as being composed of four deflected intake ducts 13 separated by four blades.

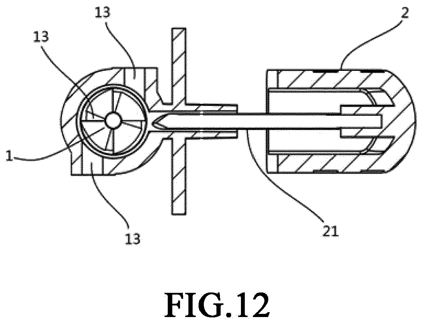

Further preferably, by referring to FIG. 11 and FIG. 12, in one embodiment, each of the bottom and side walls of the capsule chamber 1 is provided with a deflected intake passage group.

In this embodiment, each of the deflected intake duct groups at the bottom of the capsule chamber 1 and those at the side wall serve to provide deflected airflow, wherein the deflected intake duct groups at the bottom of the capsule chamber 1 can also provide a bottom-up through airflow, and the object of the present invention can also be achieved.

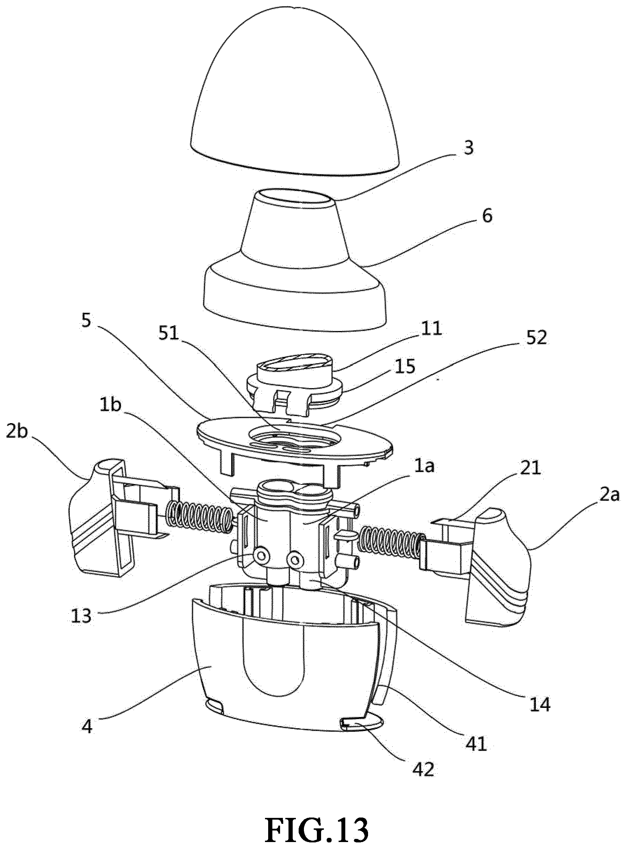

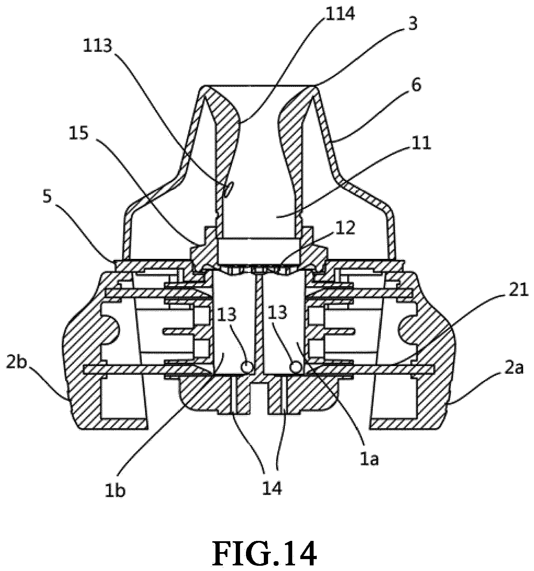

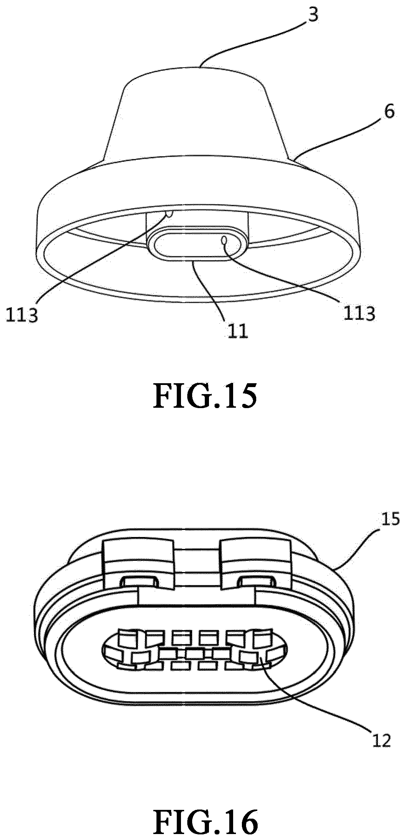

By referring to FIG. 13 and FIG. 14, it is an embodiment of a dry powder inhaler of the present disclosure, comprising: (a) capsule chambers 1 (that is, 1a and 1b in the FIG), which are cylindrical chambers that can hold a capsule upright, the top of the capsule chambers 1 is open, and the bottom of the capsule chambers 1 is provided with intake ducts 14 that ventilate with the outside air; (b) actuators 2, which comprise puncture needles 21, the puncture needles 21 are mounted for the user to operate to move toward the side walls of the capsule chambers 1 to puncture the capsules; (c) nozzle 3, illustrated in FIG. 13 and FIG. 15, which comprises an outlet duct 11 below the nozzle (for clarity, the lower part of the outlet 11 in FIG. 13 is intercepted and separated for display); wherein, the number of the capsule chambers 1 is two, the two capsule chambers 1 are arranged in parallel to form an integrally formed double-capsule chamber, and the actuators 2 are mounted individually between the capsule chambers 1, each actuator 2 is provided with two needles 21 in the height direction, a screen cover 15 is mounted at the bottom opening of the outlet duct 11, by referring to FIG. 16, a screen 12 is mounted in the screen cover 15 and can be separately connected to the top of the double-capsule chamber, so that the screen 12 covers the tops of the two capsule chambers 1.

In this embodiment, the double capsule-chamber is composed of first capsule chamber 1a and second capsule chamber 1b which are closely arranged, and the first actuator 2a and the second actuator 2b are arranged at both ends of the line where the first capsule chamber 1a and the second capsule chamber 1b are located, the first actuator 2a and the second actuator 2b can move from both sides to the middle so as to puncture the capsules in the first capsule chamber 1a and the second capsule chamber 1b, respectively.

The user separates the screen cover 15 from the top of the double-capsule chamber first, to make the top of the double-capsule chamber open; after filling the capsule chambers 1 with capsules containing different active ingredients, the screen cover 15 is closed to make the screen 12 cover the top of the capsule chambers 1 again; then, the user operates the actuators 2 to move from both sides to the middle so as to puncture the capsules in the first capsule chamber 1a and the second capsule chamber 1b, respectively, and the actuators 2 are reset by elastic components commonly used in the prior art; finally, the user covers the mouth to the nozzle 3 closely and inhales forcefully, and the outside air enters the capsule chambers through the intake duct 14 at the bottom of the capsule chambers 1, so that the capsules vibrate and rotate against the screen 12 to release the powder. The released powder from the capsules passes through the screen 12 into the outlet duct 11 and finally enters the human body.

Although the actuators 2 of this embodiment move from both sides to the middle so as to puncture the capsules in the first capsule chamber 1a and the second capsule chamber 1b, respectively, person skilled in the art can adjust the arrangement of the actuators, for example , the actuators 2 are provided on one side of the line where the first capsule chamber 1a and the second capsule chamber 1b are located, the actuators 2 comprise at least two needles in the width direction, so that when operated, the actuators 2 puncture the capsules in the first capsule chamber 1a and the second capsule chamber 1b at the same time.

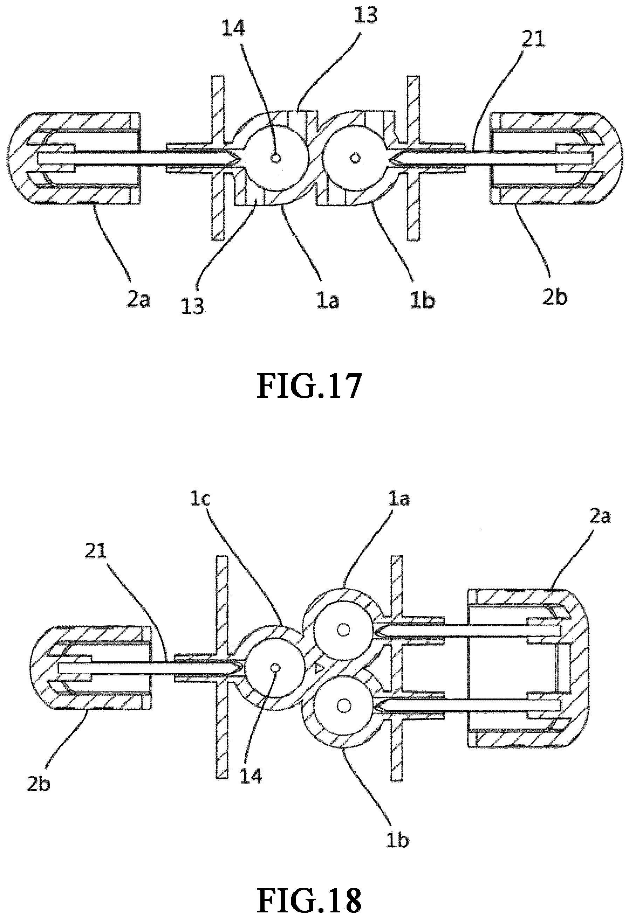

Further preferably, by referring to FIG. 17, in one embodiment, the side wall of each capsule chamber 1 is respectively provided with a deflected intake duct group. The deflected intake duct group comprises two deflected intake ducts 13 which are arranged around the central axis of the capsule chamber 1, simultaneously deflect clockwise or counterclockwise, in order to provide a spiral airflow that flows upward from the deflected intake ducts 13 when the user inhales.

Compared with the foregoing embodiment, the inhaler of this embodiment is provided with a deflected intake duct group on the side wall of the capsule chamber 1 to provide a spiral airflow that moves upward from the deflected intake duct 13 when the user inhales. The airflow can help the capsules rotate and vibrate more smoothly to release the inhalable powder, which greatly reduces the requirement of the user's inhalation flow when the capsules rotate and release medicine, and can release the powder easier.

Further preferably, by referring to FIG. 13 and FIG. 14, in one embodiment, the deflected intake duct group is provided at the lower part of the side wall of each capsule chamber 1.

Compared with the previous embodiment, the inhaler of this embodiment provides a deflected intake duct group at the lower part of the capsule chamber 1, which can not only provide a spiral airflow moving upward from the deflected intake duct 13, but also assist the bottom of the intake duct 14 to make the top of the capsule rotate against the screen 12 at the top of the capsule chambers.

Further preferably, by referring to FIG. 17, in one embodiment, the direction of the deflected intake ducts 13 is tangent to the side wall of each capsule chamber 1, so that the spiral airflow can smoothly drive the capsules to rotate.

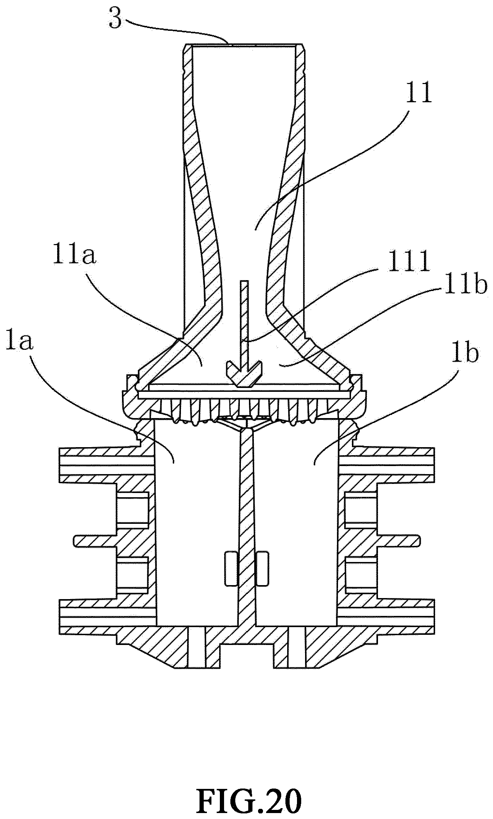

Further preferably, by referring to FIG. 20, in one embodiment of the present invention, the lower part of the outlet duct 11 is divided by a central baffle 111 to form a first sub-duct 11a and a second sub-duct 11b, which are respectively connected to the tops of the first capsule chamber and the second capsule chamber, cross-sections of the first sub-duct 11a and the second sub-duct 11b are first narrowed from the bottom to top toward the central baffle 111, and then remain unchanged, so that when the user inhales, the airflows in the first capsule chamber 1a and the second capsule chamber 1b are directed along the first sub-duct 11a and the second sub-duct 11b to merge in the upper part of the outlet duct 11 respectively.

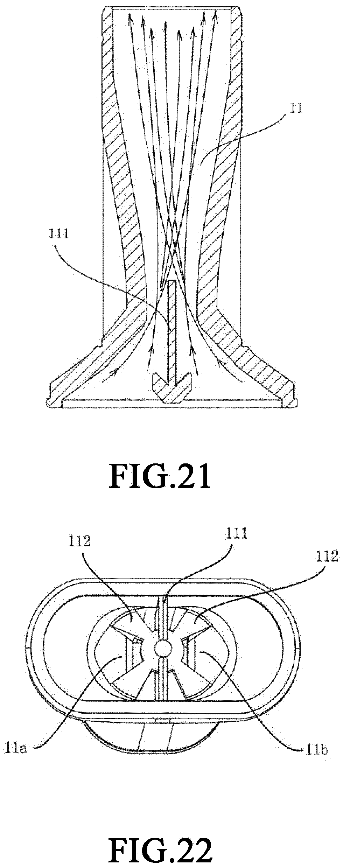

By referring to FIG. 21, through the special design of the lower part of the outlet duct 11 of this embodiment, when the user inhales, the airflows in the two capsule chambers can be gathered in the area above the central baffle 111 and converged, and then be directed to flow above the outlet duct, so not only can the drug powder released in the two capsule chambers be fully mixed, but also can the collision with the duct be reduced, thereby the resistance when user inhales can be reduced.

More preferably, by referring to FIG. 20 and FIG. 21, in one embodiment, the cross-section of the outlet duct 11 gradually increases from the top of the central baffle 111 toward the nozzle 3 and then remains unchanged. when the user inhales, the airflows in the two capsule chambers(1a, 1b) can be gathered in the area above the central baffle 111 and converged, and then be directed to flow above the outlet duct, therefore, the cross-section of the outlet duct 11 of the present embodiment gradually increases from the top of the central baffle 111 toward the nozzle 3 and then remains unchanged, which is advantageous for air to flow upwards more smoothly, as the obstruction of the of the inner wall of the airflow duct 11 to the airflow is reduced and the collision of the drug powder in the airflow with the inner wall of the outlet duct 11 is reduced. It can be understood that, in order to achieve the above effect, the cross-section of the outlet duct 11 from the top of the central baffle 111 toward the nozzle 3 does not necessarily gradually increase and then remains unchanged, as long as it does not decrease.

More preferably, by referring to FIG. 22, in one embodiment, the first sub-duct 11a and the second sub-duct 11b further comprise two sub-baffles 112, and the first sub-duct 11a and the second sub-duct 11b are further divided by the sub-baffles 112 into a plurality of narrow ducts that gradually narrow from the top of each capsule chamber from bottom to top toward the central baffle 111 respectively, the height of the sub-baffle 112 is lower than that of the central baffle 111. The cross-section of the sub-baffles is X-shaped, which is mirror-symmetrical with the central baffle 111 as the symmetry plane.

In this embodiment, the sub-ducts (11a, 11b) are further divided into six narrow ducts, each narrow duct can guide the direction of the gathered airflow more precisely according to its position, so as to make the airflows from the two capsule chambers (1a, 1b) gather more smoothly and converge on the upper area of the central baffle 111, and then be directed to flow above the outlet duct.

More preferably, by referring to FIG. 20, in one embodiment, the airflows from the first capsule chamber 1a and the second capsule chamber 1b flows along the first sub-channel 11a and the second sub-channel 11b to the upper part of the outlet duct 11 respectively when the user inhales, and the nozzle 3 is provided at a position where the airflows are sufficiently converged, and the height of the outlet duct 11 is 31 mm.

The height of the outlet duct 11 in this embodiment refers to the distance from the top of the capsule chamber to the nozzle 3, as shown in FIG. 21, in the vicinity of the nozzle at the end of the outlet duct, the airflow in the two capsule chambers (1a, 1b) has been fully converged, and the drug powder in the airflow is also fully mixed. Further increasing the distance of the outlet duct will increase the inhalation resistance.

Further preferably, by referring to FIG. 13, FIG. 14 and FIG. 15, in one embodiment of the present disclosure, the dry powder inhaler further comprises:
(d) the lower casing 4, which defines a cavity , the tope of which is open and is used for accommodating the double-capsule chamber, and the lower casing 4 is provided with two gaps 41 on the side thereof, so that at least one part of the each actuator 2 is provided located outside of the dry powder inhaler for user to operate, the gaps 41 extend downward and widen with respect to the size of the actuators 2 to provide an air intake 42, so that the inside of the cavity can ventilate with the outside air through the air intake 42; (e) the adapter plate 5 which covers the top of the lower casing 4, and a hollow port 51 is provided at the top of the multi-capsule chamber, and a screen cover 15 is detachably mounted to the hollow port 51 so that the screen 12 covers the top of each capsule chamber 1;
(f) the upper casing 6, which extends downward from the top of the nozzle 3, defines a cavity that is open at the bottom and surrounds the outlet duct 11, and covers the adapter plate 5 when the screen cover 15 is mounted at the hollow port.

First, the user separates the lower casing 4 and the upper casing 6 so as to separate the screen cover 15 from the top of the double-capsule chamber, then fills each capsule chambers 1 with capsules containing two different active ingredients, and then closes the upper casing 6 and the lower casing 4 so that the screen 12 covers the top of the capsule chambers 1 again; then, the user operates parts of the actuators 2 located out of the casings to puncture the capsules in the capsule chambers 1, the actuators 2 are reset by the elastic components commonly used in the prior art; finally, the user closely fits the mouth to the nozzle 3 and inhales in force, the outside air enters the cavity through the air intake hole 42 of the lower casing 4, and enters the capsule chambers 1 from the intake ducts 14 at the bottom of the capsule chambers 1, so that the capsules vibrate and rotate against the screen 12 to release the powder. The released powder from the capsule enters the outlet duct 11 through the screen 12 and finally enters the human body.

Compared with the previous embodiment, the dry powder inhaler of this embodiment adds the upper casing 6, the lower casing 4 and the adapter plate 5 on the premise of supporting the technical solution of the previous embodiment, thereby increasing the structural firmness of the dry powder inhaler, and making it easy to operate. The upper casing 6, the lower casing 4 and the adapter plate 5 are all conventional components of the same type of dry powder inhaler in the prior art. In this embodiment, the upper casing 6, the lower casing 4 and the adapter plate 5 are also conventional designs in the prior art.

Further preferably, by referring to FIG. 13, FIG. 14 and FIG. 15, in one embodiment, a slit 52 is provided at the junction of the upper casing 6 and the adapter plate 5, so that the internal cavity can ventilate with the outside air through the slit 52. The side wall of the outlet duct 11 is provided with a small hole 113, which is opened in a direction not facing the central axis of the outlet duct 11 to promote airflow rotate in the outlet duct 11 when the user inhales.

When the user inhales, external air can enter the internal cavity of the upper casing 6 through the slit and enter the outlet duct 11 from the small hole 113 of the outlet duct 11 to promote the rotation of the airflow in the outlet duct 11, in this embodiment, after the capsule medicine powder in each capsule chamber 1 is released, it is transmitted in the outlet duct 11 and fully mixed by rotation, so that the moving speed of the airflow arriving at the nozzle 3 is proper and the ingredients are uniform.

Further preferably, by referring to FIG. 14, in one embodiment, the diameter of the outlet duct 11 gradually decreases from bottom to top, and a neck 114 is formed before reaching the outlet, so that the moving speed of the airflow arriving at the nozzle 3 is proper and the ingredients are uniform.

By referring to FIG. 18, it is another embodiment of the dry powder inhaler of the present disclosure.

The multi-capsule chamber is composed of a first capsule chamber 1a, a second capsule chamber 1b, and a third capsule chamber 1c, which are closely arranged into a triangle. The first actuator 2a is arranged on one side of a line where the first capsule chamber 1a and the second capsule chamber 1b are connected, and the first actuator 2a is provided with two needles 21 in the width direction, and the two needles can move to the multi-capsule chamber to puncture the capsules in the first capsule chamber 1a and the second capsule chamber 1b at the same time, and the second actuator 2b is arranged on a side of the third capsule chamber 1c away from the first capsule chamber 1a and the second capsule chamber 1b, and is movable in a direction perpendicular to the straight line where the first capsule chamber 1a and the second capsule chamber 1b lie to puncture the capsule in the third capsule chamber 1c.

This embodiment provides a medicine dispenser containing three active components (or a mixture thereof) in a separated manner by providing three capsule chambers, and an intake duct 14 is provided at the bottom of each capsule chamber 1, and no deflected intake duct group is provided on the side or bottom of each capsule chamber. The arrangement of other components is the same as or similar to that in other embodiments, and details are not described herein again.

Further preferably, by referring to FIG. 18, in one embodiment, the diameter of the bottom intake duct 14 of one capsule chamber 1 is different from that of the other two capsule chambers, so that the intake airflow rate of the capsule chamber is different from that of the other capsule chambers.

In some cases, each component of the combined product needs to achieve a specific particle distribution to maximize its effect. Since each component of the present invention is released separately in each corresponding capsule chamber 1, by adjusting structural characteristics such as the size, position, opening angle, and / or the number of the intake ducts 13, the intake ducts 14, and / or the air outlet duct 11, different aerodynamic parameters can be set for each capsule chamber 1, in order to maximize the therapeutic effect of each active ingredient under the premise of simultaneous administration. In this embodiment, the size of the intake duct 14 at the bottom of the capsule chambers 1 is adjusted to give different air flow rates to affect the particle distribution of the powder in the capsules.

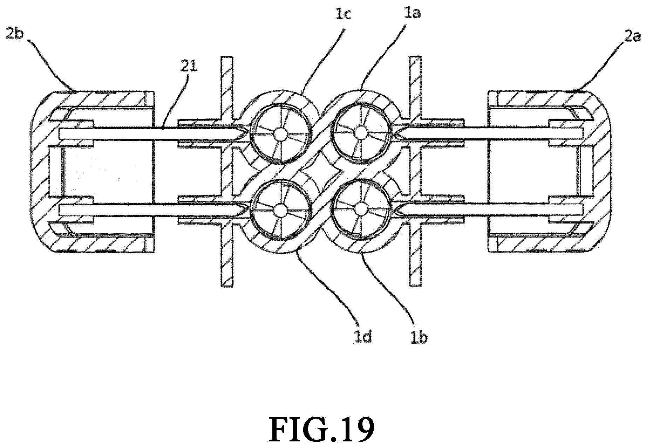

By referring to FIG. 19, it is another embodiment of the dry powder inhaler of the present disclosure,
the multi-capsule chamber 1 is composed of the first capsule chamber 1a, the second capsule chamber 1b, the third capsule chamber 1c and the fourth capsule chamber 1d, which are arranged closely as a square. The first actuator 2a and the second actuator 2b are arranged on the central axis of the square and can move from both sides to the middle, the first actuator 2a and the second actuator 2b comprises at least two needles 21 in the width direction so that the first actuator 2a simultaneously punctures the capsules in the first capsule chamber 1a and the second capsule chamber 1b, and the second actuator 2b simultaneously punctures the capsules in the third capsule chamber 1c and the fourth capsule chamber 1d.

This embodiment provides a medicine dispenser containing four active components (or a mixture thereof) in a separated manner by providing four capsule chambers. Providing four capsule chambers results in a higher requirement for the inhaler for the patient's inspiratory flow. In order to rotate and vibrate the capsule fully to release the inhalable powder, each capsule chamber 1 of this embodiment is provided with a deflected intake duct group at the bottom of the capsule chamber, and the intake duct group is arranged as a fixed impeller to provide a spiral air flow from bottom to top when the user inhales, which effectively promotes the capsule's rotation and vibration to release the inhalable powder. The specific shape of the impeller structure is shown in FIG. 10.

The above are only the specific embodiments of the present invention and are not intended to limit the scope of the present invention. Any equivalent change, modification and combination may be made whilst falling within the scope of the appended claims.

## Claims

1. Dry powder inhaler, comprising:
capsule chambers (1), which are cylindrical chambers that can hold the capsule upright, the top of the capsule chambers (1) is open, and the bottom and/or the side walls of the capsule chambers (1) are provided with intake ducts ventilating with the outside air;
actuators (2), comprising puncture needles (21), mounted for the user to operate to move toward the side walls of the capsule chambers (1) to puncture the capsules;
a nozzle (3), comprising an outlet duct (11), said outlet duct being positioned under the nozzle (3);
wherein, the number of the capsule chambers (1) is two, and all the capsule chambers (1) are arranged in parallel to form an integral multi-capsule chamber, the actuators (2) are mounted individually or in common among the capsule chambers (1), and the actuators (2) are mounted with needles (21) in the width direction of the actuators (2), at least the number of the needles (21) is the same as that of the matching capsule chambers (1), a screen cover (15), said screen cover being mounted at the bottom of the outlet duct (11) under the nozzle (3), and a screen (12), said screen being fixed in the screen cover (15) and separately connected to the top of the multi-capsule chamber, such that the screen (12) covers the top of all of the capsule chambers (1),
**characterized in that**:
the dry powder inhaler further comprises a central baffle (111), the central baffle being arranged such that
the lower part of the outlet duct (11) is divided by said central baffle (111) to form two sub-ducts, which are respectively connected to the top of each capsule chamber (1), a cross-section of each sub-duct being such that it first narrows from the top of each capsule chamber
(1) from bottom to top toward the central baffle (111), and then remains unchanged,
wherein the sub-duct comprises one or more sub-baffles (112), the one or more sub-baffles (112) dividing the sub-duct into narrower ducts, which gradually converge from bottom to top from the top of each capsule chamber toward the central baffle.

2. The dry powder inhaler of claim 1, wherein each capsule chamber (1) is provided at its bottom and/or side wall with one deflected intake duct group , the deflected intake duct group comprising two deflected intake ducts (13) which are arranged around a central axis of the capsule chamber (1), and are arranged to simultaneously deflect clockwise or
counterclockwise.

3. The dry powder inhaler of claim 2, wherein the two deflected intake ducts (13) of the deflected intake duct group of each capsule chamber (1) have the same shape and size, and are evenly arranged around a central axis of the capsule chamber (1).

4. The dry powder inhaler of claim 2 or 3, wherein the intake ducts are provided on the capsule chamber (1) in the following manner:
a direct intake duct is provided at the bottom of the capsule chamber (1), and a deflected intake duct group is provided at the side wall of the capsule chamber, or,
no intake duct is provided at the bottom of the capsule chamber (1), but a deflected intake duct group is provided in the lower part of the side wall of the capsule chamber.

5. The dry powder inhaler of any one of claims 2 to 4, wherein the deflected intake ducts (13) have an opening
on the side wall of the capsule chamber (1), said opening having a long-strip
shape, which is arranged longitudinally along the side wall.

6. The dry powder inhaler of any one of claims 1 to 5, wherein the diameter of the outlet duct (11) gradually decreases from bottom to top, and a narrow neck is formed before arriving at the nozzle (3), two small holes ventilating with the outside air being
provided under the narrow neck of the outlet duct, the small holes being symmetrically
opened around the central axis of the outlet duct (11).

7. The dry powder inhaler any one of claims 1 to 5, wherein the cross-section of the outlet duct (11) is such that it
gradually increases in a direction from the top of the central baffle (111) toward the nozzle (3).

8. The dry powder inhaler of claim 1, wherein the height of the sub-baffles (112) is lower than that of the central baffle (111).

9. The dry powder inhaler of any one of claims 1 to 8, wherein the number of capsule chambers (1) is two, the capsule chambers (1) comprising
a first capsule chamber (1a) and a second capsule chamber (1b) which are closely arranged, a first actuator (2a) and a second actuator (2b) being
arranged at both ends of a line where the first capsule chamber (1a) and the second capsule chamber (1b) are located, the first actuator (2a) and the second actuator (2b) being configured to
move from both sides to the middle so as to puncture the capsules in the first capsule chamber (1a) and the second capsule chamber (1b), respectively.

10. The dry powder inhaler of claim 9, wherein the shape of the cross-section of the sub-baffles (112) is X-shaped, taking the central baffle (111) as the plane mirror symmetry.

11. The dry powder inhaler of any one of claims 1 to 10, wherein the air resistance of the dry powder inhaler is 0.02-0.05KPa ^{0.5} minutes/liter.

12. The dry powder inhaler of claim 11, wherein the air resistance of the dry powder inhaler is 0.0325KPa ^{0.5} minutes/liter.

## Patentansprüche

1. Trockenpulverinhalator, umfassend:
Kapselkammern (1), die zylindrische Kammern sind, die die Kapsel aufrecht halten können, wobei die Oberseite der Kapselkammern (1) offen ist und die Unterseite und/oder die Seitenwände der Kapselkammern (1) mit Ansaugkanälen zur Belüftung mit der Außenluft versehen sind;
Betätigungsvorrichtungen (2), die Punktionsnadeln (21) umfassen und montiert sind, damit der Benutzer sie bedienen kann, um in Richtung der Seitenwände der Kapselkammern (1) zu bewegen, um die Kapseln zu punktieren;
eine Düse (3), die einen Auslasskanal (11) umfasst, wobei der Auslasskanal unter der Düse (3) positioniert ist;
wobei die Anzahl der Kapselkammern (1) zwei beträgt und alle Kapselkammern (1) parallel angeordnet sind, um eine integrale Mehrfachkapselkammer zu bilden, wobei die Betätigungsvorrichtungen (2) individuell oder gemeinsam inmitten der Kapselkammern (1) montiert sind und die Betätigungsvorrichtungen (2) mit Nadeln (21) in der Breitenrichtung der Betätigungsvorrichtungen (2) montiert sind, wobei mindestens die Anzahl der Nadeln (21) die gleiche ist wie die der zugehörigen Kapselkammern (1), eine Siebabdeckung (15), wobei die Siebabdeckung an der Unterseite des Auslasskanals (11) unter der Düse (3) montiert ist, und ein Sieb (12), wobei das Sieb in der Siebabdeckung (15) befestigt und derart separat mit der Oberseite der Mehrfachkapselkammer verbunden ist, dass das Sieb (12) die Oberseite aller Kapselkammern (1) bedeckt,
**dadurch gekennzeichnet, dass**
der Trockenpulverinhalator weiter eine zentrale Prallwand (111) umfasst, wobei die zentrale Prallwand derart angeordnet ist, dass der untere Teil des Auslasskanals (11) durch die zentrale Prallwand (111) unterteilt wird, um zwei Unterkanäle zu bilden, die jeweils mit der Oberseite jeder Kapselkammer (1) verbunden sind, wobei ein Querschnitt jedes Unterkanals derart ist, dass er sich zunächst von der Oberseite jeder Kapselkammer (1) von unten nach oben in Richtung der zentralen Prallwand (111) verengt und dann unverändert bleibt,
wobei der Unterkanal eine oder mehrere Unterprallwände (112) umfasst, wobei die eine oder mehreren Unterprallwände (112) den Unterkanal in schmalere Kanäle unterteilen, die von unten nach oben von der Oberseite jeder Kapselkammer in Richtung der zentralen Prallwand allmählich zusammenlaufen.

2. Trockenpulverinhalator nach Anspruch 1, wobei jede Kapselkammer (1) an ihrer Unterseite und/oder Seitenwand mit einer umgelenkten Ansaugkanalgruppe versehen ist, wobei die umgelenkte Ansaugkanalgruppe zwei umgelenkte Ansaugkanäle (13) umfasst, die um eine zentrale Achse der Kapselkammer (1) herum angeordnet sind und angeordnet sind, um gleichzeitig im Uhrzeigersinn oder gegen den Uhrzeigersinn umzulenken.

3. Trockenpulverinhalator nach Anspruch 2, wobei die zwei umgelenkten Ansaugkanäle (13) der umgelenkten Ansaugkanalgruppe jeder Kapselkammer (1) die gleiche Form und Größe aufweisen und gleichmäßig um eine zentrale Achse der Kapselkammer (1) herum angeordnet sind.

4. Trockenpulverinhalator nach Anspruch 2 oder 3, wobei die Ansaugkanäle an der Kapselkammer (1) in folgender Weise bereitgestellt sind:
ein direkter Ansaugkanal ist an der Unterseite der Kapselkammer (1) bereitgestellt und eine umgelenkte Ansaugkanalgruppe ist an der Seitenwand der Kapselkammer bereitgestellt, oder
an der Unterseite der Kapselkammer (1) ist kein Ansaugkanal bereitgestellt, aber in dem unteren Teil der Seitenwand der Kapselkammer ist eine umgelenkte Ansaugkanalgruppe bereitgestellt.

5. Trockenpulverinhalator nach einem der Ansprüche 2 bis 4, wobei die umgelenkten Ansaugkanäle (13) eine Öffnung an der Seitenwand der Kapselkammer (1) aufweisen, wobei die Öffnung die Form eines langen Streifens aufweist, der entlang der Seitenwand in Längsrichtung angeordnet ist.

6. Trockenpulverinhalator nach einem der Ansprüche 1 bis 5, wobei der Durchmesser des Auslasskanals (11) von unten nach oben allmählich abnimmt und bevor die Düse (3) erreicht wird eine schmale Verengung gebildet ist, wobei unter der schmalen Verengung des Auslasskanals zwei kleine Löcher zur Belüftung mit der Außenluft bereitgestellt sind, wobei die kleinen Löcher symmetrisch um die zentrale Achse des Auslasskanals (11) herum geöffnet sind.

7. Trockenpulverinhalator nach einem der Ansprüche 1 bis 5, wobei der Querschnitt des Auslasskanals (11) derart ist, dass er in einer Richtung von der Oberseite der zentralen Prallwand (111) in Richtung der Düse (3) allmählich zunimmt.

8. Trockenpulverinhalator nach Anspruch 1, wobei die Höhe der Unterprallwände (112) geringer ist als die der zentralen Prallwand (111).

9. Trockenpulverinhalator nach einem der Ansprüche 1 bis 8, wobei die Anzahl der Kapselkammern (1) zwei beträgt, wobei die Kapselkammern (1) eine erste Kapselkammer (1a) und eine zweite Kapselkammer (1b) umfassen, die nah beieinander angeordnet sind, wobei eine erste Betätigungsvorrichtung (2a) und eine zweite Betätigungsvorrichtung (2b) an beiden Enden einer Linie angeordnet sind, wo sich die erste Kapselkammer (1a) und die zweite Kapselkammer (1b) befinden, wobei die erste Betätigungsvorrichtung (2a) und die zweite Betätigungsvorrichtung (2b) konfiguriert sind, um sich von beiden Seiten zu der Mitte zu bewegen, um die Kapseln in der ersten Kapselkammer (1a) bzw. der zweiten Kapselkammer (1b) zu punktieren.

10. Trockenpulverinhalator nach Anspruch 9, wobei die Form des Querschnitts der Unterprallwände (112) X-förmig ist, wobei die zentrale Prallwand (111) als die Spiegelsymmetrie der Ebene genommen wird.

11. Trockenpulverinhalator nach einem der Ansprüche 1 bis 10, wobei der Luftwiderstand des Trockenpulverinhalators 0,02-0,05 KPa^{0,5} Minuten/Liter beträgt.

12. Trockenpulverinhalator nach Anspruch 11, wobei der Luftwiderstand des Trockenpulverinhalators 0,0325 KPa^{0,5} Minuten/Liter beträgt.

## Revendications

1. Inhalateur de poudre sèche, comprenant :
des chambres à capsules (1), qui sont des chambres cylindriques qui peuvent maintenir la capsule verticalement, le sommet des chambres à capsules (1) est ouvert, et le fond et/ou les parois latérales des chambres à capsules (1) sont pourvues de conduits d'admission ventilés avec l'air extérieur ;
des actionneurs (2), comprenant des aiguilles de ponction (21), montés pour que l'utilisateur puisse les actionner afin de les déplacer vers les parois latérales des chambres à capsules (1) pour perforer les capsules ;
une buse (3), comprenant un conduit de sortie (11), ledit conduit de sortie étant positionné sous la buse (3) ;
dans lequel le nombre de chambres à capsules (1) est de deux, et toutes les chambres à capsules (1) sont disposées en parallèle pour former une chambre multi-capsules intégrale, les actionneurs (2) sont montés individuellement ou en commun parmi les chambres à capsules (1), et les actionneurs (2) sont montés avec des aiguilles (21) dans le sens de la largeur des actionneurs (2), au moins le nombre d'aiguilles (21) est le même que celui des chambres à capsules correspondantes (1), un couvercle d'écran (15), ledit couvercle d'écran étant monté au fond du conduit de sortie (11) sous la buse (3), et un écran (12), ledit écran étant fixé dans le couvercle d'écran (15) et relié séparément au sommet de la chambre multi-capsules, de sorte que l'écran (12) recouvre le sommet de toutes les chambres à capsules (1),
**caractérisé en ce que**
l'inhalateur de poudre sèche comprend en outre un déflecteur central (111), le déflecteur central étant disposé de telle sorte que la partie inférieure du conduit de sortie (11) est divisée par ledit déflecteur central (111) pour former deux sous-conduits, qui sont respectivement reliés au sommet de chaque chambre à capsules (1), une section transversale de chaque sous-conduit étant telle qu'elle se rétrécit d'abord du sommet de chaque chambre à capsules (1) de bas en haut vers le déflecteur central (111), puis reste inchangée,
dans lequel le sous-conduit comprend un ou plusieurs sous-déflecteurs (112), les un ou plusieurs sous-déflecteurs (112) divisant le sous-conduit en conduits plus étroits, qui convergent progressivement de bas en haut depuis le sommet de chaque chambre de capsules vers le déflecteur central.

2. Inhalateur de poudre sèche selon la revendication 1, dans lequel chaque chambre à capsules (1) est pourvue, au niveau de son fond et/ou de sa paroi latérale, d'un groupe de conduits d'admission déviés, le groupe de conduits d'admission déviés comprenant deux conduits d'admission déviés (13) qui sont disposés autour d'un axe central de la chambre à capsules (1), et sont disposés pour dévier simultanément dans le sens des aiguilles d'une montre ou dans le sens inverse des aiguilles d'une montre.

3. Inhalateur de poudre sèche selon la revendication 2, dans lequel les deux conduits d'admission déviés (13) du groupe de conduits d'admission déviés de chaque chambre à capsules (1) présentent la même forme et la même taille, et sont disposés uniformément autour d'un axe central de la chambre à capsules (1).

4. Inhalateur de poudre sèche selon la revendication 2 ou 3, dans lequel les conduits d'admission sont prévus sur la chambre à capsules (1) de la manière suivante :
un conduit d'admission direct est prévu au niveau du fond de la chambre à capsules (1), et un groupe de conduits d'admission déviés est prévu au niveau de la paroi latérale de la chambre à capsules, ou,
aucun conduit d'admission n'est prévu au niveau du fond de la chambre à capsules (1), mais un groupe de conduits d'admission déviés est prévu dans la partie inférieure de la paroi latérale de la chambre à capsules.

5. Inhalateur de poudre sèche selon l'une quelconque des revendications 2 à 4, dans lequel les conduits d'admission déviés (13) présentent une ouverture sur la paroi latérale de la chambre à capsules (1), ladite ouverture présentant une forme de longue bande, qui est disposée longitudinalement le long de la paroi latérale.

6. Inhalateur de poudre sèche selon l'une quelconque des revendications 1 à 5, dans lequel le diamètre du conduit de sortie (11) diminue progressivement de bas en haut, et un col étroit est formé avant d'arriver à la buse (3), deux petits trous de ventilation avec l'air extérieur étant prévus sous le col étroit du conduit de sortie, les petits trous étant ouverts symétriquement autour de l'axe central du conduit de sortie (11).

7. Inhalateur de poudre sèche selon l'une quelconque des revendications 1 à 5, dans lequel la section transversale du conduit de sortie (11) est telle qu'elle augmente progressivement dans une direction allant du sommet du déflecteur central (111) vers la buse (3).

8. Inhalateur de poudre sèche selon la revendication 1, dans lequel la hauteur des sous-déflecteurs (112) est inférieure à celle du déflecteur central (111).

9. Inhalateur de poudre sèche selon l'une quelconque des revendications 1 à 8, dans lequel le nombre de chambres à capsules (1) est de deux, les chambres à capsules (1) comprenant une première chambre à capsules (1a) et une seconde chambre à capsules (1b) qui sont disposées étroitement, un premier actionneur (2a) et un second actionneur (2b) étant disposés aux deux extrémités d'une ligne où se trouvent la première chambre à capsules (1a) et la seconde chambre à capsules (1b), le premier actionneur (2a) et le second actionneur (2b) étant configurés pour se déplacer des deux côtés vers le milieu de manière à perforer les capsules dans la première chambre à capsules (1a) et la seconde chambre à capsules (1b), respectivement.

10. Inhalateur de poudre sèche selon la revendication 9, dans lequel la forme de la section transversale des sous-déflecteurs (112) est en forme de X, prenant le déflecteur central (111) comme symétrie de miroir plan.

11. Inhalateur de poudre sèche selon l'une quelconque des revendications 1 à 10, dans lequel la résistance à l'air de l'inhalateur de poudre sèche est de 0,02 à 0,05 kPa^{0,5} minutes/litre.

12. Inhalateur de poudre sèche selon la revendication 11, dans lequel la résistance à l'air de l'inhalateur de poudre sèche est de 0,0325 kPa^{0,5} minutes/litre.
